# EUROPEAN PATENT SPECIFICATION

(11) **EP 2 902 060 B1**
(45) Date of publication and mention of the grant of the patent: **28.09.2016**
(21) Application number: 15152648.0
(22) Date of filing: 27.01.2015
(51) Int. Cl.: A61M 5/315, B29C 33/42, B29C 43/18, B29C 43/20

(54) **MEDICAL GASKET MANUFACTURING METHOD**
MEDIZINISCHES DICHTUNGSHERSTELLUNGSVERFAHREN
PROCÉDÉ DE FABRICATION DE JOINT MÉDICAL

(30) Priority: 29.01.2014 JP 2014014698
(43) Date of publication of application: 05.08.2015
(73) Proprietor: Sumitomo Rubber Industries, Ltd., Kobe-shi, Hyogo-ken (JP)
(72) Inventor: Iwano, Shinya, Kobe-shi,, Hyogo 651-0072 (JP)
(74) Representative: Manitz, Finsterwald & Partner GbR

(56) References cited:
- EP-A2- 2 565 006
- US-A1- 2003 205 838
- US-A1- 2004 099 994
- US-A1- 2005 212 222

## Description

### BACKGROUND OF THE INVENTION

### Field of the Invention

The present invention relates to a medical gasket manufacturing method that includes a punching step of punching a molded sheet for separating a gasket from the molded sheet so as to reduce generation of flaws on an outer circumferential surface of the gasket.

### Description of the Background Art

In recent years, from the standpoint that medical accidents such as mistaking of medicines can be prevented, prefilled syringes obtained by syringes (injectors) being prefilled with liquid medicine, are often used. In the prefilled syringe, liquid medicine is hermetically sealed between a gasket disposed in an outer cylinder of the syringe, and a cap disposed at a nozzle portion for attaching an injection needle. When the liquid medicine is administered, an injection needle is attached to the nozzle portion, and a syringe plunger is pushed to connect to the gasket and is slid forward, thereby administrating injection solution.

As a gasket manufacturing method, a method, as described in, for example, Japanese Laid-Open Patent Publication No. 2013-49236, in which a molded sheet is formed by vacuum press molding with the use of molds, and gaskets are then separated from the molded sheet, is used.

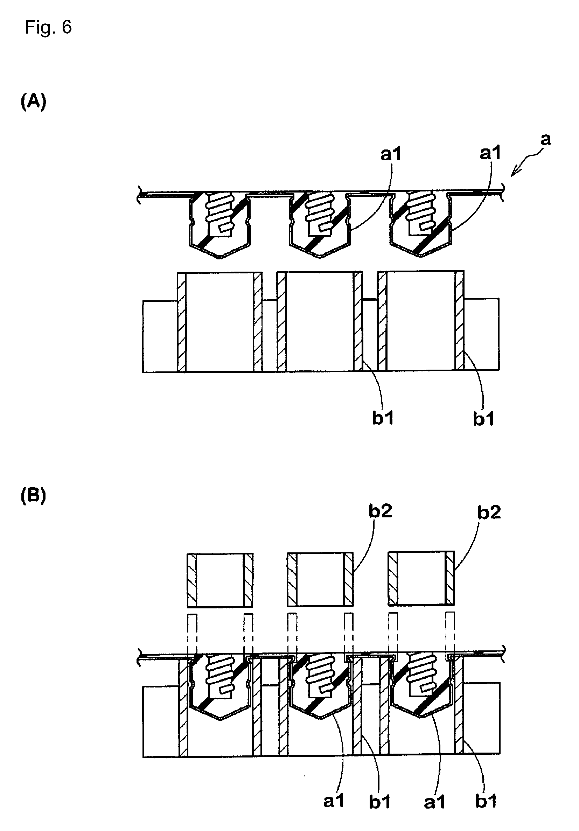

Further, as shown in FIGS. 6 (A) and (B), gaskets al of a molded sheet a are each inserted into a lower punching blade b1, positioned, and set therein, and an upper punching blade b2 is then moved down, thereby separating the gaskets al from the molded sheet a.

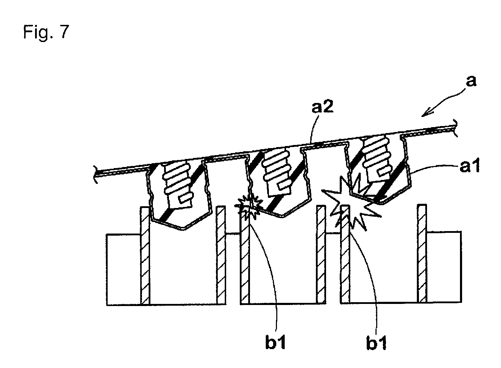

However, as shown in FIG 7, since the molded sheet a has little rigidity, the gasket al tends to be inserted diagonally into the lower punching blade b1 when the molded sheet a is set. At this time, a blade edge of the punching blade b 1 may contact with the outer circumferential surface of the gasket al, to cause flaw, thereby degrading hermetic sealing between the outer cylinder of the syringe and the gasket.

In particular, when the molded sheet a is such that rear end portions of the gaskets al are connected to each other via a sheet-like joint portion a2, projection of each gasket al from the joint portion a2 is increased. Therefore, the gasket al is more likely to contact with the blade edge, and generation of flaws becomes more significant.

US 2004/099994 A1 describes a method for producing piston stoppers that are connected by a flange. The arrangement of piston stoppers and flange is removed from a forming tool in which the arrangement is produced and placed on a blanking device to separate each piston stopper from the flange surrounding it.

### SUMMARY OF THE INVENTION

Therefore, an object of the present invention is to provide a medical gasket manufacturing method in which a plunger attaching hole of a gasket is used as a positioning portion so as to enable generation of flaws on an outer circumferential surface of the gasket to be reduced.

The object is satisfied by a method in accordance with the features of claim 1. Preferred embodiments of the present invention are described in the dependent claims.

The present invention is directed to a medical gasket manufacturing method using a plunger attaching hole in which a head portion of a plunger of a syringe is mounted, the plunger attaching hole being formed at a rear end of a gasket body that is slidable in a syringe outer cylinder, and the medical gasket manufacturing method includes:
a molded sheet forming step of forming a molded sheet in which rear end portions of a plurality of gaskets aligned with each other are connected to each other via a sheet-like joint portion; and
a punching step of punching the molded sheet to separate each gasket from the molded sheet, and
the punching step includes
a positioning step of positioning the molded sheet by a positioning pin being inserted into each plunger attaching hole of the molded sheet, and
a punching operation step of separating each gasket of the molded sheet having been positioned, from the joint portion, by using a punching blade.

In the medical gasket manufacturing method according to the present invention, the positioning pin preferably has a diameter greater than that of the plunger attaching hole.

In the medical gasket manufacturing method according to the present invention, the positioning pin preferably includes a pin body and a seat that are concentric with each other, and the pin body can be inserted into the plunger attaching hole, and the seat receives a rear end of each gasket.

In the medical gasket manufacturing method according to the present invention, the molded sheet is preferably disposed so as to face the plunger attaching hole downward, and the punching step preferably includes a removing step of removing the positioning pin from each gasket having been separated, by the positioning pin being moved downward of an upper end of a lower punching blade.

According to the present invention, as described above, the punching step of punching the molded sheet to separate each gasket from the molded sheet includes the positioning step of inserting the positioning pin into each plunger attaching hole of the molded sheet. In the positioning step, each gasket can be stably held at a precise position in an upright state.

Therefore, when punching is performed by using the punching blade, contact between the outer circumferential surface of the gasket and the punching blade can be reduced. Therefore, generation of flaws and reduction in hermetic sealing between the gasket and the syringe outer cylinder due to the flaws are less likely to occur.

### BRIEF DESCRIPTION OF THE DRAWINGS

FIG. 1 (A) is a cross-sectional view of an example of a gasket formed by a manufacturing method according to the present invention;
FIG. 1 (B) is a cross-sectional view of an example of a prefilled syringe using the gasket;
FIG. 2 (A) is a cross-sectional view illustrating a molded sheet forming step;
FIG. 2 (B) is a cross-sectional view illustrating the molded sheet forming step;
FIG. 3 is a cross-sectional view illustrating a positioning step;
FIG. 4 is a partially enlarged view illustrating the positioning step;
FIG. 5 (A) is a partially enlarged view illustrating a punching operation step;
FIG. 5 (B) is a partially enlarged view illustrating a removing step;
FIG. 6 (A) is a cross-sectional view illustrating a conventional punching step;
FIG. 6 (B) is a cross-sectional view illustrating the conventional punching step; and
FIG. 7 is a cross-sectional view illustrating a problem of the conventional step.

### DESCRIPTION OF THE PREFERRED EMBODIMENTS

Hereinafter, an embodiment of the present invention will be described in detail.

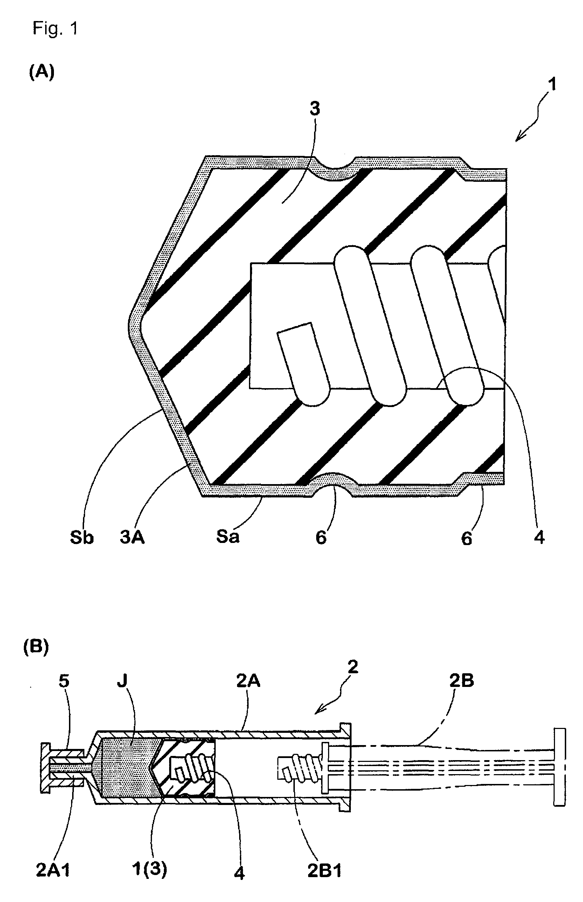

FIG. 1 (A) is a cross-sectional view of an example of a gasket 1 formed by a manufacturing method according to the present invention, and FIG. 1 (B) is a cross-sectional view of an example of a prefilled syringe 2 using the gasket 1. As shown in FIG. 1, a plunger attaching hole 4 into which a head portion 2B1 of a syringe plunger 2B is inserted is formed in the gasket 1 at a rear end of a gasket body 3 which is slidable in a syringe outer cylinder 2A.

A liquid medicine J is hermetically sealed, in the prefilled syringe 2, between the gasket 1 and a cap 5 disposed at a nozzle portion 2A1 for attaching an injection needle. When the liquid medicine is administered, the head portion 2B1 of the syringe plunger 2B is inserted into the plunger attaching hole 4 of the gasket 1 and connected to the gasket 1. As the plunger attaching hole 4, a screw hole into which the head portion 2B1 is screwed, is preferably used.

An outer circumferential surface Sa of the gasket body 3 forms a sliding surface that is slidable with respect to an inner circumferential surface of the syringe outer cylinder 2A and that hermetically contacts therewith. In this example, in order to reduce sliding resistance, at least one circumferential groove 6 is formed in the outer circumferential surface Sa so as to extend in the circumferential direction. Further, the outer circumferential surface Sa and a front end surface Sb of the gasket body 3 are coated with a coating layer 3A which is formed from an inactive resin film.

The coating layer 3A is formed in order to improve chemical resistance and reduce sliding resistance, and may be preferably formed from, for example, at least one fluororesin selected from the group consisting of tetrafluoroethylene-ethylene copolymers (ETFE), polytetrafluoroethylene (PTFE), and polychlorotetrafluoroethylene (PCTFE), and/or an olefinic resin such as ultra-high-molecular-weight polyethylene (UHMWPE). A thickness of the coating layer 3A preferably ranges, for example, from 5 µm to 150 µm.

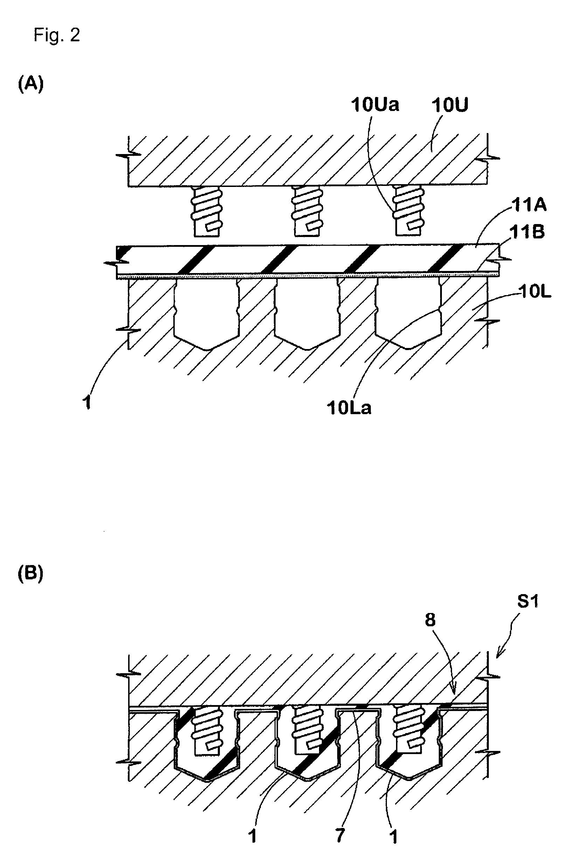

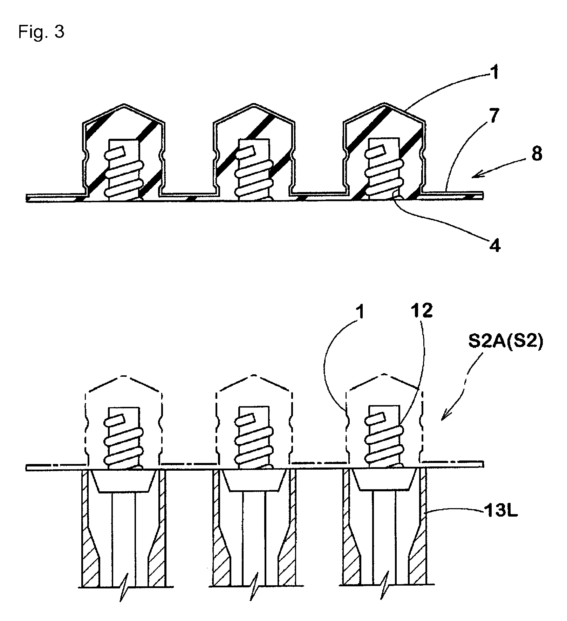

Next, a method for manufacturing the gasket 1 will be described. The manufacturing method of the present invention includes
(1) a molded sheet forming step S1 of forming a molded sheet 8 in which rear end portions of a plurality of the gaskets 1 aligned with each other are connected to each other via the sheet-like joint portion 7 (shown in FIG. 2), and
(2) a punching step S2 of punching the molded sheet 8 to separate each gasket 1 from the molded sheet 8 (shown in FIGS. 3 to 5).

As shown in FIGS. 2 (A) and (B), in the molded sheet forming step S1, vacuum press molding with the use of a pair of (upper and lower in this example) molds 10U and 10L is preferably used. Specifically, an unvulcanized rubber sheet 11A and an inactive resin film 11B are set one on top of the other between the molds 10U and 10L, and are heated and compressed by the vacuum pressing. By the heating and compressing, the rubber sheet 11A and the resin film 11B are vulcanized and adhered to each other, to form the molded sheet 8 in which the plurality of the gaskets 1 are integrally connected to each other via the joint portion 7. Recesses 10La are formed in one mold, that is, in the (lower in this example) mold 10L so as to correspond to an outer contour of each gasket 1. Projections 10Ua are formed in the other mold, that is, in the (upper in this example) mold 10U so as to correspond to an outer contour of each plunger attaching hole 4.

Further, the punching step S2 includes a positioning step S2A using positioning pins 12, and a punching operation step S2B using annular punching blades 13U and 13L disposed on one surface side and the other surface side of the molded sheet 8.

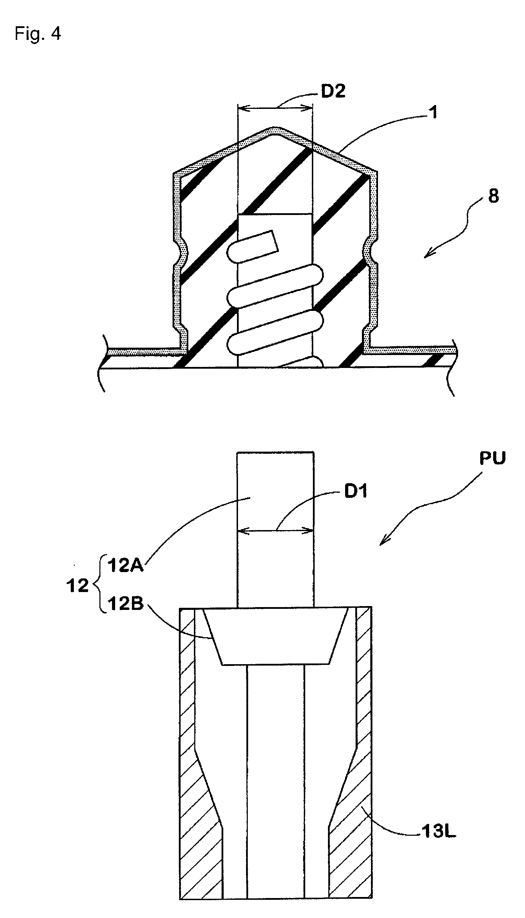

As shown in FIGS. 3 and 4, in the positioning step S2A, the positioning pins 12 are inserted into the plunger attaching holes 4, respectively, of the molded sheet 8, to position the molded sheet 8. Thus, the gaskets 1 of the molded sheet 8 are held at precise positions in an upright state. In this example, the molded sheet 8 is held such that the plunger attaching holes 4 face downward, and the positioning pins 12 are inserted into the plunger attaching holes 4 from below.

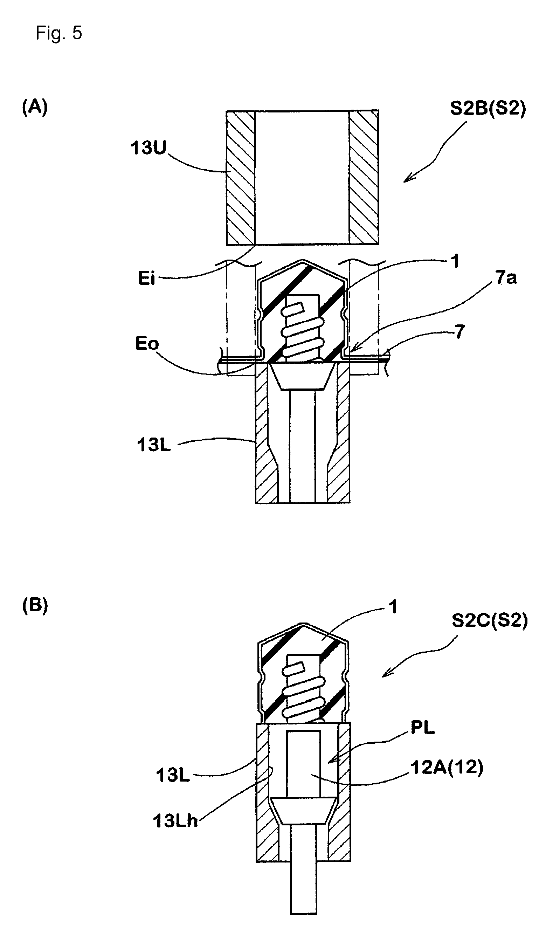

Each positioning pin 12 is disposed, concentrically with the lower punching blade 13L, in the lower punching blade 13L. Further, each positioning pin 12 is held so as to be movable between a lower position PL (shown in FIG. 5 (B)) at which the upper end of the positioning pin 12 is located below the upper end of the lower punching blade 13L, and an upper position PU (shown in FIGS. 3 and 4) at which the upper end of the positioning pin 12 projects upward of the upper end of the lower punching blade 13L. Each positioning pin 12 preferably includes a pin body 12A and a seat 12B that are concentric with each other, and the pin body 12A can be inserted into the plunger attaching hole 4, and the seat 12B receives the rear end of the gasket 1. The seat 12B allows the gasket 1 to be held in the upright state with enhanced stability.

As shown in FIG. 5 (A), in the punching operation step S2B, each gasket 1 of the molded sheet 8 which has been positioned, is separated from the joint portion 7 by using the upper and lower punching blades 13U and 13L. In this example, the upper punching blade 13U is moved down to cut the joint portion 7 between an outer edge Eo of the lower punching blade 13L and an inner edge Ei of the upper punching blade 13U. At this time, the joint portion 7 is cut such that a root portion 7a of the joint portion 7 is drawn. Therefore, a portion of the joint portion 7 which is left uncut is extremely small. Therefore, in a case where, as in this example, the circumferential groove 6 has a groove bottom at the rear end of the gasket 1 and the joint portion 7 extends from the groove bottom, the portion of the joint portion 7 which is left uncut can be put in the circumferential groove 6, whereby the uncut portion can be prevented from protruding from the outer circumferential surface Sa of the gasket 1.

Further, as shown in FIG. 5 (B), the punching step S2 includes a removing step S2C of removing the positioning pin 12 from the gasket 1 which has been separated in the punching operation step S2B. The removing step S2C is performed by the positioning pin 12 being moved down to the lower position PL. The diameter of an inner hole 13Lh of the lower punching blade 13L is less than the outer diameter of the gasket 1. Therefore, the gasket 1 having been separated does not fall into the lower punching blade 13L.

Thus, since the punching step S2 includes the positioning step S2A in which the positioning pin 12 is inserted into the plunger attaching hole 4, each gasket 1 can be held at the precise position in the vertically upright state. As a result, in the punching operation step S2B, generation of flaws due to contact of the outer circumferential surface Sa of the gasket 1 with the upper punching blade 13U can be reduced.

A diameter D1 (shown in FIG. 4) of the pin body 12A is preferably greater than an inner diameter D2 of the plunger attaching hole 4. In a case where D1≤D2 is satisfied, rattling occurs between the pin body 12A and the plunger attaching hole 4, and the gasket 1 becomes unstable, thereby causing reduction of positioning accuracy. Therefore, sufficient prevention of generation of flaws becomes difficult. In this viewpoint, the diameter D1 is preferably greater than or equal to 105% of the inner diameter D2, and is more preferably greater than or equal to 110% thereof. On the other hand, when the diameter D1 is excessively great, the pin body 12A cannot be inserted into the plunger attaching hole 4, or the gasket 1 is expanded and a flaw is likely to be generated due to contact with the upper punching blade 13U even if the pin body 12A can be inserted. In this viewpoint, the diameter D1 is preferably not greater than 125% of the inner diameter D2, and is more preferably not greater than 120% thereof. The inner diameter D2 of the plunger attaching hole 4 represents a diameter of a perfect circle that can be inscribed in the attaching hole 4. For example, in the case of a screw hole, the inner diameter D2 corresponds to an inner diameter at a pilot hole portion (a portion having no thread groove).

Although the particularly preferred embodiments of the present invention have been described above in detail, the present invention is not limited to the above embodiments shown in the drawings, and various modifications can be made to implement the present invention.

### [Examples]

A molded sheet was punched so as to separate each gasket from the molded sheet in each punching step indicated by the specifications in Table 1, and comparison in each of generation of flaws and liquid leakage among the obtained gasket, was performed.

Each molded sheet was formed according to the molded sheet forming step shown in FIG. 2 as follows. That is, an unvulcanized rubber sheet (formed from a chlorinated butyl rubber: JIS A hardness after vulcanization was 60 degrees), and an inactive resin film (PTFE film: the thickness thereof was 70 µm) were set one on top of the other between upper and lower molds, and were heated and compressed at 160°C for 15 minutes by vacuum pressing, to form each molded sheet.
(1) Generation of flaws:
   The outer circumferential surface of each gasket was examined by using an appearance examining camera machine. A product including a flaw having a length of 0.5 mm or greater, was determined as a defective product. 1000 gaskets were examined and evaluated to obtain an incidence of the defective products. The less the numerical value is, the better the result is.
(2) Liquid leakage:
   In compliance with "Mekkin-zumi chusha-tou kijun (standards for sterile injection syringes)" (Notified on December 11, 1998 by Director of the Pharmaceutical and Medical Safety Bureau, the Ministry of Health and Welfare in Iyakuhatsu No. 1079), experiments were executed as follows.
   ·A jig having a length greater than that of a plunger attaching hole (screw hole) of each gasket was fitted into the plunger attaching hole, and the gasket was placed so as to face its liquid contact side upward. A syringe outer cylinder (injection syringe) was inserted straight to plug the syringe. As the syringe outer cylinder, a cylinder having a capacity of 1 mL and an inner diameter of 6.3 mm, and formed from COP resin as a material of the syringe, was used.
   ·Subsequently, water colored with methylene blue was charged through a nozzle portion to a position of a scale mark corresponding to 3/4 of the nominal capacity.
   ·A nozzle cap and a plunger were attached. The syringe outer cylinder was faced downward, and a predetermined pressure (490 kPa) was applied to the plunger for 10 seconds, and the syringe was left untouched for one day. Thereafter, the syringe was observed at 10-fold magnification. 20 syringes were examined for leakage of the water into circumferential grooves of the gaskets.

The evaluation criteria are as follows.
A: No leakage was observed.
B: Slight linear leakage was observed.
C: Leakage was clearly observed.

**[Table 1]**

| | | Conventional art | Example 1 | Example 2 | Example 3 |
|---|---|---|---|---|---|
| <Punching step> | | FIG. 6 | FIGS. 3 to 5 | | |
| Positioning step | | Not executed | Executed | | |
| · Positioning pin | | ---- | Used | | |
| ··· Ratio D1/D2 in diameter | | ---- | 100 | 105 | 115 |
| ···Seat | | ---- | Not used | Used | Used |
| | | | | | |
| Generation of flaws | | 2.0% | 0.5% | 0.1% | 0.0% |
| Liquid leakage | A | 19/20 | 20/20 | 20/20 | 20/20 |
| | B | 1/20 | 0/20 | 0/20 | 0/20 |
| | C | 0/20 | 0/20 | 0/20 | 0/20 |

As indicated in the table, it can be confirmed that generation of flaws and liquid leakage for the gasket have been improved in Examples.

## Claims

1. A medical gasket manufacturing method using a plunger attaching hole (4) in which a head portion of a plunger (2B) of a syringe (2) is mounted, the plunger attaching hole (4) being formed at a rear end of a gasket body (3) that is slidable in a syringe outer cylinder (2A), the medical gasket manufacturing method comprising:
a molded sheet forming step (S1) of forming a molded sheet (8) in which rear end portions of a plurality of gaskets (1) aligned with each other are connected to each other via a sheet-like joint portion (7); and
a punching step (S2) of punching the molded sheet (8) to separate each gasket (1) from the molded sheet (8), wherein
the punching step (S2) includes
a positioning step (S2A) of positioning the molded sheet (8) by a positioning pin (12) being inserted into each plunger attaching hole (4) of the molded sheet (8), and
a punching operation step (S2B) of separating each gasket (1) of the molded sheet (8) having been positioned, from the joint portion (7), by using a punching blade (13U, 13L).

2. The medical gasket manufacturing method according to claim 1, wherein the positioning pin (12) has a diameter greater than that of the plunger attaching hole (4).

3. The medical gasket manufacturing method according to claim 1 or 2, wherein the positioning pin (12) includes a pin body (12A) and a seat (12B) that are concentric with each other, and the pin body (12A) can be inserted into the plunger attaching hole (4), and the seat (12B) receives a rear end of each gasket (1).

4. The medical gasket manufacturing method according to any one of claims 1 to 3, wherein
the molded sheet (8) is disposed so as to face the plunger attaching hole (4) downward, and
the punching step (S2) includes a removing step (S2C) of removing the positioning pin (12) from each gasket (1) having been separated, by the positioning pin (12) being moved downward of an upper end of a lower punching blade (13L).

## Patentansprüche

1. Verfahren zum Herstellen einer medizinischen Dichtung unter Verwendung eines Stempelbefestigungsloches (4), in welchem ein Kopfabschnitt eines Stempels (2B) einer Spritze (2) montiert ist, wobei das Stempelbefestigungsloch (4) an einem rückwärtigen Ende eines Dichtungskörpers (3) geformt ist, der in einem Spritzenaußenzylinder (2A) verschiebbar ist, wobei das Verfahren zum Herstellen einer medizinischen Dichtung umfasst:
einen Schritt zum Ausbilden einer geformten Lage (S1) zum Ausbilden einer geformten Lage (8), in welchem rückwärtige Endabschnitte einer Mehrzahl von Dichtungen (1), die miteinander ausgerichtet sind, miteinander über einen lagenartigen Fügeabschnitt (7) verbunden werden; und
einen Stanzschritt (S2) zum Stanzen der geformten Lage (8), um jede Dichtung (1) von der geformten Lage (8) zu trennen, wobei
der Stanzschritt (S2) umfasst:
einen Positionierungsschritt (S2A) zum Positionieren der geformten Lage (8) durch einen Positionierungsstift (12), der in jedes Stempelbefestigungsloch (4) der geformten Lage (8) eingesetzt wird, und
einen Stanzbetriebsschritt (S2B) zum Trennen jeder Dichtung (1) der geformten Lage (8), die positioniert worden ist, von dem Fügeabschnitt (7) unter Verwendung einer Stanzklinge (13U, 13L).

2. Verfahren zum Herstellen einer medizinischen Dichtung nach Anspruch 1, wobei der Positionierungsstift (12) einen Durchmesser besitzt, der größer als der des Stempelbefestigungsloches (4) ist.

3. Verfahren zum Herstellen einer medizinischen Dichtung nach einem der Ansprüche 1 oder 2, wobei der Positionierungsstift (12) einen Stiftkörper (12A) und einen Sitz (12B) aufweist, die konzentrisch zueinander sind, und der Stiftkörper (12A) in das Stempelbefestigungsloch (4) eingesetzt werden kann und der Sitz (12B) ein rückwärtiges Ende jeder Dichtung (1) aufnimmt.

4. Verfahren zum Herstellen einer medizinischen Dichtung nach einem der Ansprüche 1 bis 3, wobei:
die geformte Lage (8) so angeordnet ist, dass sie zu dem Stempelbefestigungsloch (4) abwärts weist, und
der Stanzschritt (S2) einen Entfernungsschritt (S2C) zum Entfernen des Positionierungsstifts (12) von jeder Dichtung (1), die getrennt worden ist, umfasst, indem der Positionierungsstift (12) von einem oberen Ende einer unteren Stanzklinge (13L) abwärts bewegt wird.

## Revendications

1. Procédé de fabrication d'un joint médical utilisant un trou d'attache (4) pour plongeur dans lequel est montée une portion d'un plongeur (2B) d'une seringue (2), le trou d'attache (4) pour plongeur étant formé à une extrémité arrière d'un corps de joint (3) qui est capable de coulisser dans un cylindre extérieur (2A) d'une seringue,
le procédé de fabrication d'un joint médical comprenant :
une étape de formation d'une feuille moulée (S1) consistant à former une feuille moulée (8) dans laquelle des portions d'extrémité arrière d'une pluralité de joints (1) alignés les uns avec les autres sont connectées les unes aux autres via une portion de joint semblable à une feuille (7) ; et
une étape de poinçonnage (S2) consistant à poinçonner la feuille moulée (8) pour séparer chaque joint (1) depuis la feuille moulée (8),
dans lequel
l'étape de poinçonnage (S2) inclut
une étape de positionnement (S2A) consistant à positionner la feuille moulée (8) par une broche de positionnement (12) qui est insérée dans chaque trou d'attache (4) pour plongeur de la feuille moulée (8), et une étape de poinçonnage (S2B) consistant à séparer chaque joint (1) de la feuille moulée (8) qui a été positionnée, depuis la portion de joint (7), en utilisant une lame de poinçonnage (13U, 13L).

2. Procédé de fabrication d'un joint médical selon la revendication 1, dans lequel la broche de positionnement (12) a un diamètre plus grand que celui du trou d'attache (4) pour plongeur.

3. Procédé de fabrication d'un joint médical selon la revendication 1 ou 2, dans lequel la broche de positionnement (12) inclut un corps de broche (12A) et un siège (12B) qui sont concentriques l'un avec l'autre, et le corps de broche (12A) peut être inséré dans le trou d'attache (4) pour plongeur, et le siège (12B) reçoit une extrémité arrière de chaque joint (1).

4. Procédé de fabrication d'un joint médical selon l'une quelconque des revendications 1 à 3, dans lequel
la feuille moulée (8) est disposée de manière à faire face au trou d'attache (4) pour plongeur en direction du bas, et
l'étape de poinçonnage (S2) inclut une étape d'enlèvement (S2C) consistant à enlever la broche de positionnement (12) depuis chaque joint (1) qui a été séparé, par la broche de positionnement (12) qui est déplacée vers le bas, d'une extrémité supérieure d'une lame de poinçonnage inférieur (13L).
